Europäisches Patentamt

European Patent Office

Office européen des brevets

(11) Veröffentlichungsnummer: **0 453 628 A2**

# (12) EUROPÄISCHE PATENTANMELDUNG

(21) Anmeldenummer: 90119712.9

(22) Anmeldetag: 15.10.90

(51) Int. Cl.5: **A61K 7/043**

(30) Priorität: 26.04.90 DE 4013250

(43) Veröffentlichungstag der Anmeldung:
30.10.91 Patentblatt 91/44

(84) Benannte Vertragsstaaten:
CH DE FR IT LI

(71) Anmelder: Heraeus Kulzer GmbH
Heraeusstr. 12 - 14
W-6450 Hanau(DE)

(72) Erfinder: Eppinger, Bernhard
Am Kirmesplatz 17
W-6290 Weilburg(DE)
Erfinder: Krahmer, Melanie
Spessartstrasse 6
W-6393 Wehrheim(DE)
Erfinder: Heindl, Detlef, Dr.
Am Mühlberg 5
W-6294 Weinbach 3(DE)

(74) Vertreter: Grimm, Ekkehard
Heraeus Holding GmbH Heraeusstrasse 12 - 14
W-6450 Hanau/Main(DE)

(54) **Verfahren zur Herstellung von Kunststoff-Überzügen auf Fingernägeln.**

(57) Auf Fingernägeln gut haftende Kunststoff-Überzüge lassen sich mit photopolymerisierbaren Fingernagel-Präparaten auf Methacrylat-Basis erzeugen, die 1-20 Gewichts-% mindestens eines Monomethacryloyloxyäthylesters einer Dicarbonsäure enthalten.

EP 0 453 628 A2

Die Erfindung betrifft ein Verfahren zur Herstellung von Kunststoff-Überzügen auf Fingernägeln durch Auftragen eines photopolymerisierbaren Präparats auf Methacrylat-Basis und Aushärten des Präparats durch Bestrahlung mit ultra-violettem und/oder sichtbarem Licht.

Es ist bekannt, Fingernägel zur Verschönerung oder zum Schutz mit aus polymerisierbaren Präparaten erzeugten Kunststoff-Überzügen zu versehen. Die polymerisierbaren Präparate - meist als Fingernagel-Präparate bezeichnet - sollen keine den Nagel und die Nagelhaut schädigenden Bestandteile enthalten und eine gleichermaßen für das Auftragen wie auch für das Modellieren günstige Konsistenz besitzen. Die durch Polymerisation ausgehärteten Überzüge sollen mindestens für einige Tage auf dem natürlichen Nagel haften bleiben und möglichst ähnliche Eigenschaften wie der natürliche Nagel besitzen.

Anders als die erst vor Gebrauch durch Mischen zuzubereitenden selbsthärtenden Fingernagel-Präparate sind die durch Photopolymerisation auszuhärtenden Präparate unter Ausschluß von Licht lagerfähige, gebrauchsfertige Einkomponenten-Präparatemit dem Vorteil einer gleichbleibenden Konsistenz und einer längeren Verarbeitungszeit, da das Aushärten erst bei Belichtung erfolgt.

Ein photopolymerisierbares Fingernagel-Präparat ist zum Beispiel aus der US-Patentschrift 4 058 442 bekannt. Es besteht aus einer Mischung aus Mono- und Polyestern der Methacrylsäure, einem bei UV-Bestrahlung die Polymerisation auslösenden Initiator, einem Modifizierungsmittel aus einem Elastomer oder Elastomer-Cellulosederivat-Gemisch und gegebenenfalls einem feinteiligen anorganischen Füllstoff und anderen Zusätzen, wie zum Beispiel Farb- und Duftstoffen.

Um die Haftfestigkeit der Überzüge auf den Nägeln zu verbessern, wird in der veröffentlichten europäischen Patentanmeldung 325 038 eine Vorbehandlung der Nägel mit der Lösung einer haftvermittelnden Substanz aus einem ungesättigten Monoester der Maleinsäure, Dimethylmaleinsäure, Citraconsäure, Bernsteinsäure oder Glutarsäure in einem organischen Lösungsmittel vorgeschlagen.

Es ist nun die Aufgabe der Erfindung, ein Verfahren zur Herstellung von Kunststoff-Überzügen auf Fingernägeln durch Auftragen eines photopolymerisierbaren Fingernagel-Präparats auf Methacrylat-Basis und Aushärten des Präparats durch Bestrahlung mit ultraviolettem und/oder sichtbarem Licht zu finden, mit dem sich, ohne daß es der Vorbehandlung mit der Lösung einer haftvermittelnden Substanz bedarf, auf den Fingernägeln gut haftende Überzüge erzeugen lassen.

Das die Lösung der Aufgabe darstellende Verfahren ist dadurch gekennzeichnet, daß ein photopolymerisierbares Präparat aufgetragen wird, das 20-60 Gewichts-% acryliertes Polyurethan, 10-40 Gewichts-% Hydroxyäthylmethacrylat und/oder Hydroxypropylmethacrylat, 1-20 Gewichts-% Diurethandimethacrylat aus 2,2,4-Trimethylhexamethylendiisocyanat und 2-Hydroxyäthylmethacrylat, 1-15 Gewichts-% Polyäthertriacrylat, 0,3-15 Gewichts-% Acetylcellulose, 0,02-10 Gewichts-% Photopolymerisationskatalysator und 1-20 Gewichts-% mindestens eines Dicarbonsäuremonomethacryloyloxyäthylesters enthält.

Das Verfahren hat sich besonders bewährt, wenn ein photopolymerisierbares Präparat aufgetragen wird, das 30-50 Gewichts-% acryliertes Polyurethan, 20-30 Gewichts-% Hydroxyäthylmethacrylat und/oder Hydroxypropylmethacrylat, 5-15 Gewichts-% Diurethandimethacrylat aus 2,2,4-Trimethylhexamethylendiisocynat und 2-Hydroxyäthylmethacrylat, 5-10 Gewichts-% Polyäthertriacrylat, 0,5-5 Gewichts-% Acetylcellulose, 3-8 Gewichts-% Photopolymerisationskatalysator und 5-15 Gewichts-% mindestens eines Dicarbonsäuremonomethacryloyloxyäthylesters enthält.

Vorzugsweise wird für das Verfahren ein Präparat verwendet, das mindestens einen Dicarbonsäuremonomethacryloyloxyäthylester aus der Gruppe $C_{1-6}$-Alkandicarbonsäuremonomethacryloyloxyäthylester, $C_{2-6}$-Alkendicarbonsäuremonomethacryloyloxyäthylester und Benzoldicarbonsäuremonomethacryloyloxyäthylester enthält.

Besonders vorteilhafte Ausführungsformen des erfindungsgemäßen Verfahrens ergeben sich mit einem Präparat, das den Monomethacryloyloxyäthylester der Bernsteinsäure, der Maleinsäure und/oder der Phthalsäure enthält.

Als Hydroxyalkylmethacrylat wird das 2-Hydroxyäthylmethacrylat bevorzugt.

Als Photopolymerisationskatalysatoren eignen sich alle für diesen Zweck bekannten Katalysatoren, die bei Bestrahlung mit Licht im Wellenlängenbereich von 350 - 550 nm die Polymerisation des Präparats auslösen und dermatologisch unbedenklich sind, wobei Ketone und Acylphosphinoxide, wie aus der deutschen Offenlegungsschrift 28 30 927 bekannt, bevorzugt werden. Besonders bewährt haben sich 1-(4-Isopropylphenyl)-2-hydroxy-2-methylpropanon, Phenyl-2-hydroxy-2-methylpropanon, Campherchinon, Benzildimethylketal (1,2-Diphenyl-2,2-dimethoxyäthanon), 2,4,6-Trimethylbenzoyldiphenylphosphinoxid oder Gemische aus zwei dieser Verbindungen, vorzugsweise im Gewichtsverhältnis von 0,5-1,5 zu 0,5-1,5.

Mit der als Verdickungsmittel wirkenden Acetylcellulose wird eine sowohl für das Auftragen als auch das Formen und Modellieren günstige Konsistenz des Präparats erreicht.

Zusätzlich kann das Präparat noch Thixotropiermittel, wie kolloidales Siliciumdioxid, Farbund Duftstoffe und Antioxidations- und Stabilisierungsmittel enthalten. Die Menge dieser Zusätze liegt vorzugsweise im Bereich von 2-5 Gewichts-%.

Zur Herstellung der Überzüge wird das Präparat auf die gereinigten Fingernägel aufgetragen und in gewünschter Weise, gegebenenfalls unter Verwendung einer Schablone, geformt und modelliert. Das Aushärten des Präparats erfolgt durch Bestrahlung der Hand in einem Fingernagel-Bestrahlungsgerät, wie es zum Beispiel aus der deutschen Offenlegungsschrift 38 25 324 bekannt ist.

Die nach dem erfindungsgemäßen Verfahren erzeugten Kunststoff-Überzüge zeichnen sich durch ihre gute Haftfestigkeit auf den Fingernägeln aus. Auch nach längerer Tragezeit weisen sie keine Risse und Spalten auf. Da ihre Eigenschaften denen des natürlichen Nagels ähnlich sind, werden sie nicht als störend empfunden.

Mit dem Verfahren gemäß der Erfindung wird die Möglichkeit geschaffen, auf den Fingernägeln gut haftende Kunststoff-Überzüge zu erzeugen, ohne daß eine Vorbehandlung der Nägel mit der Lösung einer haftvermittelnden Substanz erforderlich ist. Mit der Vorbehandlungslösung entfallen auch durch ihre Anwendung bedingte Fehlermöglichkeiten, wie zum Beispiel zu dünnes oder zu dickes Aufbringen oder auf dem Fingernagel verbleibende Lösungsmittel-Reste, die die Qualität der Überzüge beeinträchtigen können. Daß kein ein organisches Lösungsmittel enthaltendes Präparat verwendet wird, ist ein weiterer Vorteil des Verfahrens.

Zur näheren Erläuterung wird in der Tabelle I die Zusammensetzung einiger beispielhafter Ausführungsformen des bei dem Verfahren gemäß der Erfindung einzusetzenden Präparats angegeben.

Tabelle I

| Beispiel<br>Zusammensetzung [Gew.-%] | 1 | 2 | 3 | 4 | 5 | 6 | 7 | 8 |
|---|---|---|---|---|---|---|---|---|
| Acryliertes aromatisches Polyurethan[1] | 41 | 44 | 40 | 41 | 41 | 41 | 41 | 42 |
| 2-Hydroxyäthylmethacrylat | 27 | 28 | 25 | 27 | 27 | 27 | 27 | 27 |
| Diurethandimethacrylat[2] | 9 | 10 | 8 | 9 | 9 | 9 | 9 | 9 |
| Polyäthertriacylat[3] | 4 | 4 | 4 | 4 | 4 | 4 | 4 | 4 |
| Maleinsäuremono-2-methacryloyloxyäthylester | 11 | 6 | 15 | | | 11 | 11 | 10,5 |
| Bernsteinsäuremono-2-methacryloyloxyäthylester | | | | 11 | | | | |
| Phthalsäuremono-2-methacryloyloxyäthylester | | | | | 11 | | | |
| 1-(4-Isopropylphenyl)-2-hydroxy-2-methylpropanon | 1,5 | 1,5 | 1,5 | 1,5 | 1,5 | | | |
| Phenyl-2-hydroxy-2-methylpropanon | 1,8 | 1,8 | 1,8 | 1,8 | 1,8 | 1,8 | | |
| Campherchinon | | | | | | 1,5 | 1,5 | |
| Benzildimethylketal | | | | | | | 1,8 | 1,8 |
| 2,4,6-Trimethylbenzoyldiphenylphosphinoxid | | | | | | | | 1 |
| Acetylcellulose | 0,8 | 0,8 | 0,8 | 0,8 | 0,8 | 0,8 | 0,8 | 0,8 |
| Aerosil | 1,2 | 1,2 | 1,2 | 1,2 | 1,2 | 1,2 | 1,2 | 1,2 |
| Duftstoff | 2 | 2 | 2 | 2 | 2 | 2 | 2 | 2 |
| Farbstoff | 0,4 | 0,4 | 0,4 | 0,4 | 0,4 | 0,4 | 0,4 | 0,4 |
| Stabilisator | 0,3 | 0,3 | 0,3 | 0,3 | 0,3 | 0,3 | 0,3 | 0,3 |

[1] Actilan® 18 von SNPE, Frankreich

[2] Reaktionsprodukt aus 2,2,4-Trimethylhexamethylendiiso-cyanat und 2-Hydroxyäthylmethacrylat

[3] Setacure® AM 554 von Akzo

Zur Bestimmung der Haftfestigkeit der daraus auf den Fingernägeln erzeugten Kunststoff-Überzüge wird nach einer übliche Tätigkeiten, wie Händewaschen und Geschirrspülen, einschließenden Tragezeit von 24 Stunden und von 7 Tagen mit einem Skalpell die Haftung der Überzüge am Nagelrand geprüft. Die Ergebnisse der Prüfung werden in der Tabelle II angegeben.

EP 0 453 628 A2

Tabelle II

| Beispiel | Haftfestigkeit | |
|---|---|---|
| | nach 24 h | nach 7 Tagen |
| 1 | sehr gut | sehr gut |
| 2 | gut | gut |
| 3 | gut | gut |
| 4 | sehr gut | gut |
| 5 | sehr gut | gut |
| 6 | sehr gut | sehr gut |
| 7 | sehr gut | sehr gut |
| 8 | sehr gut | gut |

sehr gut = keine Ablösung

gut      = Erweichung des Überzugrandes

**Patentansprüche**

1.  Verfahren zur Herstellung von Kunststoff-Überzügen auf Fingernägeln durch Auftragen eines photopolymerisierbaren Präparats auf Methacrylat-Basis und Aushärten des Präparats durch Bestrahlung mit ultraviolettem und/oder sichtbarem Licht, dadurch gekennzeichnet, daß ein photopolymerisierbares Präparat aufgetragen wird, das 20-60 Gewichts-% acryliertes Polyurethan, 10-40 Gewichts-% Hydroxyäthylmethacrylat und/oder Hydroxypropylmethacrylat, 1-20 Gewichts-% Diurethandimethacrylat aus 2,2,4-Trimethylhexamethylendiisocyanat und 2-Hydroxyäthylmethacrylat, 1-15 Gewichts-% Polyäthertriacrylat, 0,3-15 Gewichts-% Acetylcellulose, 0,02-10 Gewichts-% Photopolymerisationskatalysator und 1-20 Gewichts-% mindestens eines Dicarbonsäuremonomethacryloyloxyäthylesters enthält.

2.  Verfahren nach Anspruch 1, dadurch gekennzeichnet, daß ein photopolymerisierbares Präparat aufgetragen wird, das 30-50 Gewichts-% acryliertes Polyurethan, 20-30 Gewichts-% Hydroxyäthylmethacrylat und/oder Hydroxypropylmethacrylat, 5-15 Gewichts-% Diurethandimethacrylat aus 2,2,4-Trimethylhexamethylendiisocyanat und 2-Hydroxyäthylmethacrylat, 5-10 Gewichts-5 Polyäthertriacrylat, 0,5-5 Gewichts-% Acetylcellulose, 3-8 Gewichts-% Photopolymerisationskatalysator und 5-15 Gewichts-% mindestens eines Dicarbonsäuremonomethacryloyloxyäthylesters enthält.

3.  Verfahren nach Anspruch 1 oder 2, dadurch gekennzeichnet, daß das Präparat mindestens einen Dicarbonsäuremonomethacryloyloxyäthylester aus der Gruppe $C_{1-6}$-Alkandicarbonsäuremonometha-cryloyloxyäthylester, $C_{2-6}$-Alkendicarbonsäuremonomethacryloyloxyäthylester und Benzoldicarbonsäu-remonomethacryloyloxyäthylester enthält.

4.  Verfahren nach einem der Ansprüche 1 bis 3, dadurch gekennzeichnet, daß das Präparat den Monomethacryloyloxyäthylester der Bernsteinsäure enthält.

5.  Verfahren nach einem der Ansprüche 1 bis 4, dadurch gekennzeichnet, daß das Präparat den Monomethacryloyloxyäthylester der Maleinsäure enthält.

6.  Verfahren nach einem der Ansprüche 1 bis 5, dadurch gekennzeichnet, daß das Präparat den Monomethacryloyloxyäthylester der Phthalsäure enthält.

7.  Verfahren nach einem der Ansprüche 1 bis 6, dadurch gekennzeichnet, daß das Präparat 2-Hydroxy-äthylmethacrylat enthält.

4

8. Verfahren nach einem der Ansprüche 1 bis 7, dadurch gekennzeichnet, daß das Präparat als Photopolymerisationskatalysator 1-(4-Isopropylphenyl)-2-hydroxy-2-methylpropanon, Phenyl-2-hydroxy-2-methylpropanon, Campherchinon, Benzildimethylketal (1,2-Diphenyl-2,2-dimethoxyäthanon), 2,4,6-Trimethylbenzoyldiphenylphosphinoxid oder ein Gemisch aus zwei dieser Verbindungen enthält.